# EUROPÄISCHE PATENTSCHRIFT

(11) **EP 1 306 103 B1**
(45) Veröffentlichungstag und Bekanntmachung des Hinweises auf die Patenterteilung: **19.10.2005**
(21) Anmeldenummer: 02090354.8
(22) Anmeldetag: 15.10.2002
(51) Int. Cl.: A61N 1/05

(54) **Elektrodenanordnung**
Electrode assembly
Ensemble d'électrodes

(30) Priorität: 24.10.2001 DE 10153842
(43) Veröffentlichungstag der Anmeldung: 02.05.2003
(73) Patentinhaber: BIOTRONIK Mess- und Therapiegeräte GmbH & Co Ingenieurbüro Berlin, 12359 Berlin (DE)
(72) Erfinder: Kolberg, Gernot, 12043 Berlin (DE); Lenski, Hartmut, 15806 Glienick (DE); Schaldach, Max, Verstorben (DE); Kranz Curt, Dr.-Ing., 14163 Berlin (DE)
(74) Vertreter: Eisenführ, Speiser & Partner

(56) Entgegenhaltungen:
- WO-A-01/68177
- DE-A- 2 605 590
- US-A- 5 267 958
- US-A- 6 056 722
- US-A- 6 095 990

## Beschreibung

Die Erfindung betrifft eine intravaskuläre Elektrodenanordnung umfassend eine erste und zweite, für das Platzieren in Blutgefäßen wie Venen oder Arterien angepasste Elektrodenleitung mit jeweils einer Stimulations- und/oder Sensing-Elektrode im Bereich eines distalen Endes der jeweiligen Elektrodenleitung.

Im Sinne der vorliegenden Erfindung wird zwischen intrakardialen Elektrodenleitungen einerseits und intravaskulären Elektrodenleitungen in dem Sinne unterschieden, dass intrakardiale Elektrodenleitungen dazu ausgebildet sind, dass ihre Sensing- oder Stimulationselektroden nach der Implantation im Atrium oder Ventrikel eines Herzens platziert sind, während intravaskuläre Elektrodenleitungen im hier verstandenen engeren Sinne solche Elektrodenleitungen sind, deren Elektroden sich nach der Implantation in einer Ader wie beispielsweise einer Vene oder einer Arterie befinden. Aufgrund der räumlichen Enge, die in solchen Blutgefäßen herrscht, sind die entsprechenden intravaskulären Implantationsorte schwieriger anzusteuern, als intrakardiale Implantationsorte. Außerdem variieren die intravaskulären Implantationsorte von Patient zu Patient stärker, als dies bei intrakardialen Implantationsorten der Fall ist.

Im Zusammenhang mit der Stimulation eines Herzens stellt die Stimulation des linken Herzens deshalb ein Problem dar, weil eine Platzierung intrakardialer Elektroden im linken Herzen höchst aufwendig ist. Daher werden zur Stimulation des linken Atriums oder Ventrikels statt dessen intravaskuläre Elektroden verwendet, die beispielsweise in Herzkranzgefäße eingeführt werden. Damit ergibt sich das im vorangegangenen Absatz geschilderte Problem.

DE 2 605 590 beschreibt eine Elektrodenanordnung gemäß dem Oberbegriff des unabhängigen Anspruchs 1. Ein System zur Fixierung der Elektroderleitungen zueinander nach ihrer Plazierung wird ober nicht beschrieben.

Dieses Problem wird erfindungsgemäß durch eine intravaskuläre Elektrodenanordnung der zweiten Elektrodenleitung vorgesehen sind, wobei die Längsführungen derart manipulierbar ausgebildet sind, dass sie in einem ersten Zustand ein Längsverschieben zwischen den beiden Elektrodenleitungen erlauben, während der Langeführungen in einem zweiter Zustand ein Längsverschieben zwischen den beiden Elektrodenleitungen verbinden.

Dieser Lösung liegt der Gedanke zugrunde, für das linke Atrium und den linken Ventrikel eines Herzens jeweils einen eigenen intravaskulären Elektrodenkatheter vorzusehen, die jeweils hinsichtlich der unterschiedlichen Implantationsorte optimal gestaltet sind. Bevorzugt ist in diesem Zusammenhang eine Elektrodenanordnung, bei der die erste Elektrodenleitung mit den Längsführungen als atrialer Elektrodenkatheter ausgebildet ist, während die zweite Elektrodenleitung der Führung der ersten, ventrikulären Elektrodenleitung dient und als ventrikulärer Elektrodenkatheter ausgebildet ist. Zur Implantation dieser Elektrodenleitungen wird zunächst die ventrikuläre Elektrodenleitung eingeführt. Dies ist einfach auf übliche weise möglich, da die ventrikuläre Elektrodenleitung keine Führungen aufweist. Anschließend wird die atriale Elektrodenleitung mit ihren Führungen so über die ventrikuläre Elektrodenleitung geschoben, dass die ventrikuläre elektrodenleitung innerhalb der Längsführungen verläuft. Die atriale Elektrodenleitung mit ihren Führungen kann dann vorgeschoben werden, wobei sie durch die ventrikuläre Elektrodenleitung geführt wird.

Um eine möglichst präzise Führung der ersten Elektrodenleitung zu ermöglichen, weisen die Längsführungen Ösen auf, die über die zweite Elektrodenleitung zu schieben und dementsprechend bezüglich ihres Innendurchmessers an den Außendurchmesser der zweiten Elektrodenleitung angepasst sind und diesem im wesentlichen entsprechen.

Der Durchmesser der beiden Elektrodenleitungen ist vorzugsweise geringer als 2mm, damit sie als intravaskuläre Elektrodenleitungen eingesetzt werden können.

Um eine zwei Elektrodenleitungen umfassende Elektrodenanordnung der vorgenannten Art zu ermöglichen, ist in erster Linie nur eine der beiden Elektrodenleitung im erfindungsgemäßen Sinne gegenüber dem Stand der Technik abzuwandeln, während die zweite Elektrodenleitung dem Stand der Technik entsprechen kann. Dementsprechend besteht die erfindungsgemäße Lösung des eingangs genannten Problems bereits in einer einzigen, erfindungsgemäß gestalteten Elektrodenleitung mit außen an der Elektrodenleitung angebrachten Längsführungen, welche vorzugsweise Ösen umfassen.

Die Längsführungen enthalten vorzugsweise Nitinol, eine Nickel-Titanlegierung.

Die Längsführungen weisen in Längsrichtung der Elektrodenleitung zwei Enden auf, von denen vorzugsweise zumindest eines der Enden eine Schräge aufweist, die ausgehend von der Elektrodenleitung zu dem jeweils anderen Ende hin geneigt ist. Besonders bevorzugt ist eine Variante, bei der beide Enden der Längsführungen entsprechend abgeschrägt sind. Eine derartige Schräge erleichtert das Einführen der Elektrodenleitung bzw. der Elektrodenanordnung.

In einer bevorzugten Ausführungsvariante ist die Schräge der Längsführungen von einer offenen Gitterstruktur oder einem Drahtgeflecht gebildet. Diese Gitterstruktur oder das Drahtgeflecht ist insbesondere nach Art eines Stents ausgestaltet.

Bevorzugt wird auch eine Elektrodenleitung, bei der die Längsführungen auf eine Hülle der Elektrodenleitung nachträglich aufgebracht sind. Die Alternative hierzu besteht in Längsführungen, die von dem Hüllenmaterial selbst gebildet sind, so dass Längsführungen und die Hülle der Elektrodenleitung zusammen einteilig ausgebildet sind. Bei der bevorzugten Variante enthalten die Hulle der Elektrodenieitung und die Längsführungen unterschiedliches Material, beispielsweise ist die Hülle der Elektrodenleitung von silikonhaltigem Material gebildet, während die Längsführungen im wesentlichen von nitinolhaltigem Material, also einem Metall, gebildet sind.

In einer bevorzugten Ausführungsvariante weisen die Längsführungen reibungsvermindernde Zungen auf, die zwischen einem zur Aufnahme der zweiten Elektrodenleitung vorgesehenen Hohlraum der Längsführungen und der Hülle der ersten Elektrodenleitung angeordnet und derart ausgebildet sind, dass eine Hülle der zweiten Elektrodenleitung vermittels der Zungen einen Abstand zu der Hülle der ersten Elektrodenleitung hat. Mit diesen Abstand herstellenden Zungen kann die unmittelbare Reibung der Hüllen der beiden Elektrodenleitungen aneinander weitgehend vermieden werden, so dass sich die beiden Elektrodenleitungen möglichst leichtgängig relativ zueinander längsverschieben lassen. Mit Zunge ist in diesem Zusammenhang jede Art abstandsvermittelnder Bestandteil der Längsführungen gemeint, insbesondere metallhaltige Bestandteile der Längsführung, die innerhalb der Längsführungen zwischen den beiden Elektrodenleitungen angeordnet sind.

Um schließlich einen Kurzschluss oder elektrischen Kontakt der Sensing- oder Stimulationselektroden der einen Elektrodenleitung zu den Elektroden der zweiten Elektrodenleitung zu verhindern, ist es vorgesehen, insbesondere die Elektroden der ersten Elektrodenleitung auf derjenigen Seite des Umfangs der ersten Elektrodenleitung elektrisch zu isolieren, die aufgrund der Längsführungen der zweiten Elektrodenleitung zugewandt ist.

Die Erfindung soll nun anhand von Ausführungsbeispielen mit Hilfe der Figuren näher erläutert werden. Die Figuren zeigen:
- Figur 1: eine Seitenansicht einer Elektrodenanordnung mit zwei Elektrodenleitungen;
- Figur 2: den in Figur 1 eingetragenen Schnitt A-A in vergrößerter Darstellung;
- Figur 3: einen Abschnitt einer ersten Elektrodenleitung mit einer Längsführung in Form einer Nitinolhülse;
- Figur 4: eine Seitenansicht eines Abschnitts einer ersten Elektrodenleitung mit einer Längsführung, die Schrägen aufweist;
- Figur 5: einen Abschnitt einer Elektrodenleitung mit einer Variante einer Längsführung, die abstandsvermittelnde Zungen aufweist;
- Figur 6: das distale Ende einer ersten Elektrodenleitung mit teilweise isolierten Stimulations- oder Sensing-Elektroden; und
- Figur 7: eine alternative Ausführungsform ähnlich Figur 1.

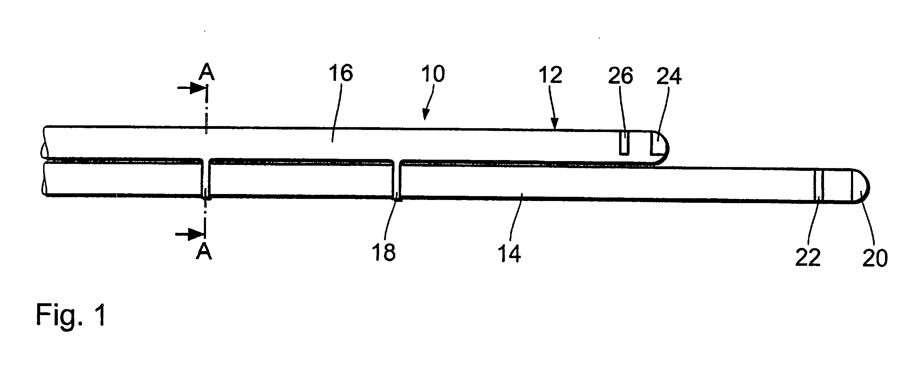

Figur 1 zeigt eine Elektrodenanordnung 10 mit einem atrialen Elektrodenkatheter 12 und einem zweiten ventrikulären Elektrodenkatheter 14. Die beiden Elektrodenkatheter 12 und 14 sind aufgrund ihres Durchmessers und ihrer Flexibilität für eine intravaskuläre Anordnung geeignet ausgebildet. Das heißt die beiden intravaskulären Elektrodenkatheter 12 und 14 sind dünn und flexibel genug, um in einem Blutgefäß wie einer Vene oder Arterie platziert zu werden.

Beide Elektrodenkatheter 12 und 14 weisen eine Hülle auf, von denen die Hülle 16 des atrialen Elektrodenkatheters 12 mit Längsführungen 18 in Form von Ösen ausgestattet ist, die zur längsverschieblichen Aufnahme des ventrikulären Elektrodenkatheters 14 ausgebildet sind. Ein geeignetes Material für die Hüllen der Elektrodenkatheter sowie für die Längsführungen 18 ist beispielsweise Silikon, welches im Bereich der Längsführungen 18 beispielsweise durch eine Versteifung aus Draht oder Ähnlichem verstärkt sein kann.

Beide Elektrodenkatheter 12 und 14 weisen an Ihrem jeweiligen distalen Ende Stimulations- und/oder Sensing-Elektroden auf. Die Stimulations- und Sensing-Elektroden 20 und 22 des ventrikulären Elektrodenkatheters 14 sind dabei in üblicher Weise als Spitzenelektrode 20 und als Ringelektrode 22 ausgebildet. Entsprechende Elektroden ermöglichen wahlweise das unipolare oder bipolare Stimulieren oder Abfühlen. Alternativ können beide Elektrodenkatheter auch nur mit einer Elektrode in Form einer Spitzen- oder Ringelektrode ausgestattet sein.

Auch der atriale Elektrodenkatheter 12 weist eine Spitzenelektrode 24 und eine Ringelektrode 26 auf, wobei diese beiden Elektroden auf ihrer der ventrikulären Elektrodenleitung 14 zugewandten Seite elektrisch isoliert sind, damit es nicht zu einem Kurzschluss zwischen den Elektroden 20 und 22 des ventrikulären Elektrodenkatheters 14 und den entsprechenden Elektroden 24 und 26 des atrialen Elektrodenkatheters 12 kommt, falls sich die distalen Enden der beiden Elektrodenkatheter beim Längsverschieben des atrialen Elektrodenkatheters bezüglich des ventrikulären Elektrodenkatheters 12 auf gleicher Höhe befinden.

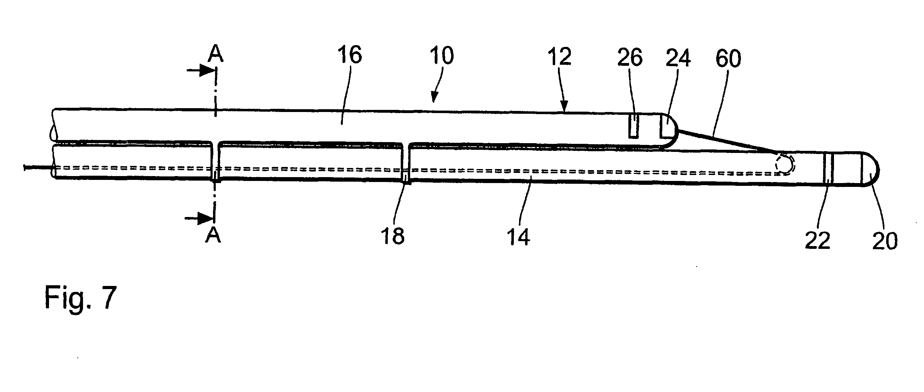

Im Falle der Implantation wird zunächst die ventrikuläre Elektrodenleitung so in Blutgefäße eines Patienten eingeführt, dass das distale Ende der Elektrodenleitung 14 mit ihren Elektroden 20 und 22 den gewünschten Zielort, z.B. im Coronar-Sinus einnimmt. Anschließend werden die Ösen 18 der atrialen Elektrodenleitung 12 am proximalen Ende der ventrikulären Elektrodenleitung 14 auf die Elektrodenleitung 14 geschoben, so dass die atriale Elektrodenleitung mittels ihrer Ösen 18 von der bereits implantierten ventrikulären Elektrodenleitung 14 geführt wird. Hat auch das distale Ende der atrialen Elektrodenleitung 12 den bestimmungsgemäßen Zielort im Atrium des Herzen erreicht, wird die atriale Elektrodenleitung 12 mittels ihrer Ösen 18 gegenüber der ventrikulären Elektrodenleitung 14 durch Verklemmen fixiert. Entsprechende Ösen 18, die ein solches Verklemmen ermöglichen, sind in Figur 7 dargestellt.

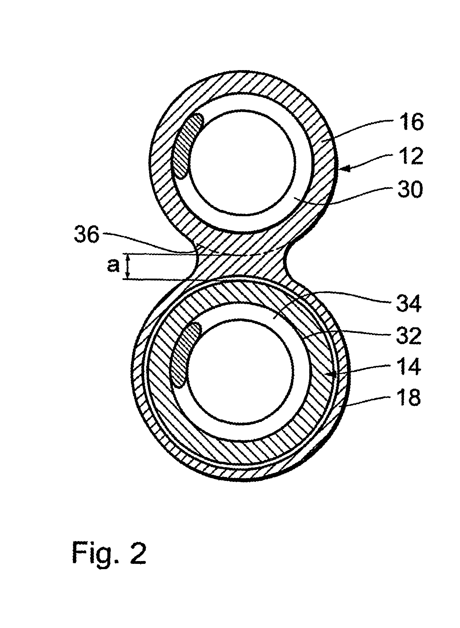

Figur 2 zeigt einen Schnitt durch die Elektrodenanordnung aus Figur 1 im Bereich einer der Längsführungen 18. Wie Figur 2 zu nehmen ist, ist die Längsführung 18 ein Bestandteil der Hülle 16 der atrialen Elektrodenleitung 12. Innerhalb dieser Hülle 16 der atrialen Elektrodenleitung 12 ist eine versteifende Helixwendel 30 aus Metall angeordnet.

Die ventrikuläre Elektrodenleitung 14 ist ähnlich aufgebaut wie die atriale Elektronenleitung 12, nur dass sie keine Längsführungen 18 aufweist. Auch die ventrikuläre Elektrodenleitung 14 weist eine Hülle 32 beispielsweise aus Silikon und eine darin angeordnete versteifende Metallwendel 34 auf. Der Außendurchmesser der Hülle 32 ist etwas geringer als der Innendurchmesser der Längsführung 18, um eine Beweglichkeit der atrialen Elektrodenleitung 12 bezüglich der ventrikulären Elektrodenleitung 14 zu erlauben.

Die Längsführung 18 ist so ausgebildet, dass die Hüllen 16 und 32 der atrialen bzw. ventrikulären der Elektrodenleitung 12 bzw. 14 einen Abstand a voneinander haben, wie dies durch die gestrichelte Linie 36 in Figur 2 angedeutet ist.

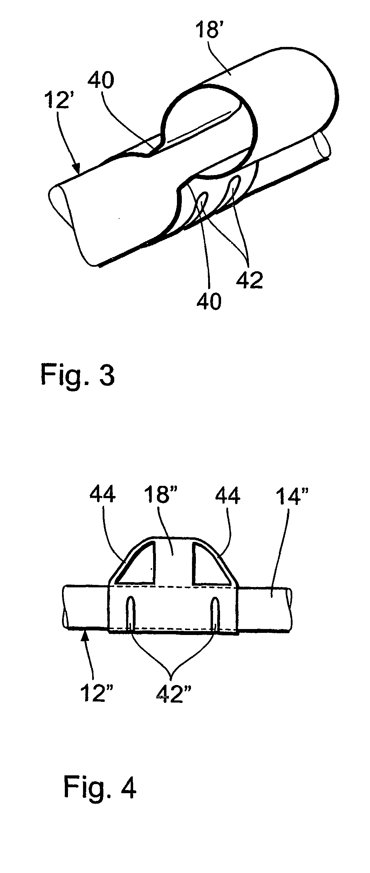

Figur 3 zeigt einen kurzen Abschnitt einer atrialen Elektrodenleitung 12' mit einer alternativen Längsführung 18', die als Metallhülse außen auf die Elektrodenleitung 12' aufgesetzt ist. Die Gestaltung der Metallhülse 18 mit Einschnürungen 40 ist so gewählt, dass eine in die Metallhülse 18' eingeführte ventrikuläre Elektrodenleitung einen seitlichen Abstand zur atrialen Elektrodenleitung 12' einnimmt. Durch Crimpen erzeugte Einschnürungen 42 an der Metallhülse 18' sorgen für einen sicheren Halt auf der atrialen Elektrodenleitungen 12'.

Figur 4 zeigt wiederum einen kurzen Abschnitt einer atrialen Elektrodenleitung 12" mit drauf angebrachter Längsführung 18". Die Längsführung 18" ist ähnlich gestaltet wie die Längsführung 18' aus Figur 3 und weist wie diese haltbringende Einschnürungen 42" auf, die beispielsweise durch Crimpen erzeugt sind. Zusätzlich weist die Längsführung 18" jedoch auch zwei Schrägen 44 auf, die nach Art eines Stents stegartig ausgebildet sind und verhindern sollen, dass sich die Längsführungen 18" beim Einführen der atrialen Elektrodenleitung 12" im Gewebe verhaken.

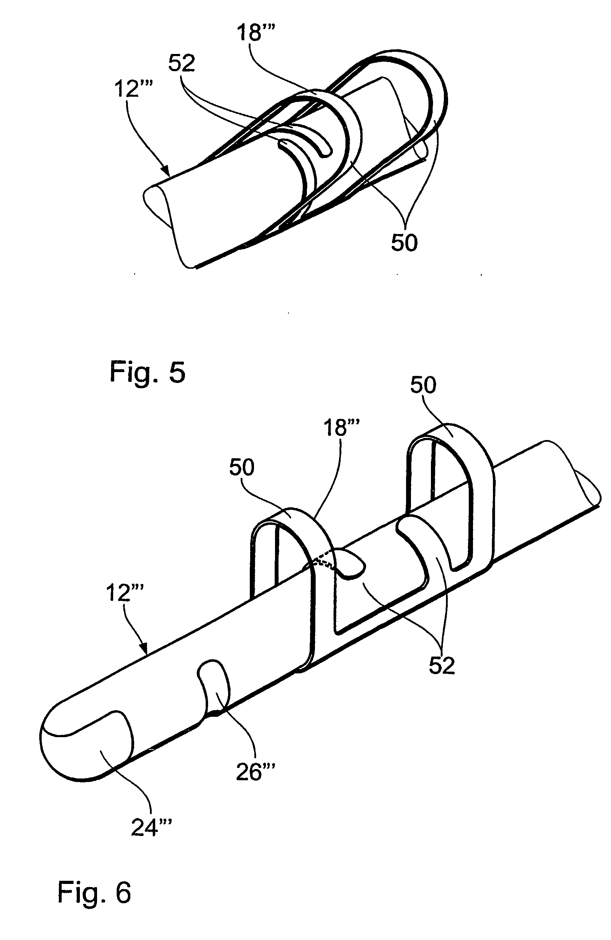

Figur 5 zeigt wiederum einen kurzen Abschnitt einer atrialen Elektrodenleitung 12"' mit darauf angeordneter Längsführung 18'''. Auch die Längsführung 18"' ist vorzugsweise aus Metall wie beispielsweise Nitinol gebildet und weist zum Halten und für die Führung entlang einer ventrikulären Elektrodenleitung zwei Bügel 50 auf. Außerdem weist die Längsführung 18''' zwei Zungen 52 auf, die um die atriale Elektrodenleitung 12''' herumgeformt sind und sich in Umfangsrichtung der atrialen Elektrodenleitung 12"' überlappen. Die Zungen 52 haben zum einen die Funktion, der Längsführung 18"' Halt auf der atrialen Elektrodenleitung 12''' zu geben, indem sie eine entsprechende Klammerwirkung entfalten. Außerdem sorgt die Dicke der Zungen 52 dafür, dass eine in der Längsführung 18''' befindliche ventrikuläre Elektrodenleitung einen Abstand von der atrialen Elektrodenleitung 12''' hat. Daher können bei der Längsführung 18''' solche Einschnürungen wie die Einschnürungen 40 der Längsführung 18' aus Figur 3 entfallen.

Figur 6 zeigt das distale Ende einer atrialen Elektrodenleitung 12'''. mit einer Längsführung 18"', wie sie bereits in Figur 5 beschrieben ist. Aus Figur 6 geht hervor, dass die Spitzenelektrode 24''' und die Ringelektrode 26''' auf derjenigen Umfangsseite des atrialen Elektrodenkatheters 12''' isoliert sind, die beim Implantieren einer ventrikulären Elektrodenleitung zugewandt ist und an dieser vorbeigeführt wird. Diese Umfangsrichtung wird durch die Ausrichtung der Längsführung 18''' bestimmt. Die Ausrichtung der Längsführung 18''' bezüglich der atrialen Elektrodenleitung 12''' mit ihren teilweise isolierten Elektroden 24''' und 26''' ist somit so zu wählen, dass die Bügel 50 die Längsführung 18''' in diejenige Umfangsrichtung weisen, in der die Elektroden 24''' und 26''' der atrialen Elektrodenleitung 12''' isoliert sind.

Gemäß der in Figur 7 abgebildeten Variante ist gegenüber der in Figur 1 dargestellten Variante zusätzlich ein Seilzug 60 vorgesehen, der innerhalb der ventrikulären Elektrodenleitung verläuft und an deren distalem Ende austritt. Dieser Seilzug ist mit dem distalen Ende der atrialen Elektrodenleitung verbunden, so dass es möglich ist, das distale Ende der atrialen Elektrodenleitung mit Hilfe des Seilzuges in Richtung des distalen Ende der ventrikulären Elektrodenleitung zu Ziehen. Der Seilzug verlauft zu diesem Zweck innerhalb der ventrikulären Elektrodenleitung bis zu deren proximalem Ende, um von dort aus betätigt zu werden.

In einer nicht dargestellten Variante einer atrialen Elektrodenleitung mit einer Längsführung bzw. Öse, kann die Öse gegenüber der Längsachse der atrialen Elektrodenleitung z.B. mittels eines Seilzuges so gekippt werden, dass es zu einem Verklemmen der Öse mit einer in der Öse befindlichen ventrikulären Elektrodenleitung kommt, so dass beiden Elektrodenleitungen relativ zueinander fixiert sind.

Alternativ hierzu kann die Öse an der atrialen Elektrodenleitung auch anderweitig verformbar ausgebildet sein, um ein Verklemmen mit einer Elektrodenleitung in der Öse zu ermöglichen.

Außerdem ist es möglich, die atriale Elektrodenleitung im Bereich der Öse z.B. S-förmig oder anderweitig gebogen derart vorzuformen, dass die Längsachsen der atrialen Elektrodenleitung und der ventrikulären Elektrodenleitung im Bereich der Öse dazu neigen, nicht parallel zueinander zu verlaufen, so dass es zu Verspannungen zwischen der Öse und der in ihr befindlichen zweiten Elektrodenleitung kommt. Während des Einführens der ersten, atrialen Elektrodenleitung wird dann deren Vorformung z.B. mittels eines entsprechenden Mandrins aufgehoben, so dass atriale und ventrikuläre Elektrodenleitung leicht relativ zueinander längsverschieblich sind. Anschließend - wenn die atriale Elektrodenleitung die vorgesehene Position eingenommen hat - wird der Mandrin entfernt, die atriale Elektrodenleitung verkantet aufgrund ihrer Vorformung die Öse mit der ventrikulären Elektrodenleitung und die beiden Elektrodenleitung sind relativ zueinander fixiert.

## Patentansprüche

1. Intravaskuläre Elektrodenanordnung (10) umfassend eine erste und eine zweite, für das Plazieren in Blutgefäßen wie Venen oder Arterien angepaßte Elektrodenleitung (12, 14) mit jeweils wenigstens einer Stimulations- und/oder Sensing-Elektrode im Bereich des distalen Endes der jeweiligen Elektrodenleitung, sowie mit außen an der ersten Elektrodenleitung (12, 12', 12", 12"') angebrachte Längsführungen (18, 18', 18", 18''') für die zweite Elektrodenleitung (14), **dadurch gekennzeichnet, dass** die Längsführungen (18, 18', 18", 18''') derart manipulierbar ausgebildet sind, dass sie in einem ersten Zustand ein Längsverschieben der Elektrodenleitung (12) und einer zweiten Elektrodenleitung (14) zwischen der Elektrodenleitung (12) und einer zweiten Elektrodenleitung (14) erlauben, während die Längsführungen (18, 18', 18", 18"') in einem zweiten Zustand ein Längsverschieben zwischen den beiden Elektrodenleitungen (12, 14) verhindern und aus diese Weise die beiden Elektrodenleitungen (12, 14) relativ zueinander fixieren.

2. Elektrodenanordnung nach Anspruch 1, **dadurch gekennzeichnet, dass** die Längsführungen (18, 18', 18", 18"') Ösen mit einem Innendurchmesser umfassen, welcher an den Außendurchmesser der zweiten Elektrodenleitung (14) angepasst ist und diesem im wesentlichen entspricht.

3. Elektrodenanordnung nach Anspruch 1, **dadurch gekennzeichnet, dass** der Außendurchmesser der ersten und der zweiten Elektrodenleitung (12, 14) geringer ist als 2 mm.

4. Elektrodenanordnung nach Anspruch 1, **dadurch gekennzeichnet, dass** die erste Elektrodenleitung (12, 12', 12", 12''') als atrialer Elektrodenkatheter ausgebildet ist und dass die zweite Elektrodenleitung (14) als ventrikulärer Elektrodenkatheter ausgebildet ist.

5. Elektrodenleitung für eine Elektrodenanordnung nach einem der Ansprüche 1 bis 5, **gekennzeichnet durch** außen an der Elektrodenleitung (12, 14) angebrachte Längsführungen (18. 18', 18", 18'''), die derart manipulierbar ausgebildet sind, dass sie in einem ersten Zustand ein Längsverschieben der Elektrodenleitung (12) und einer zweiten Elektrodenleitung (14) zwischen den beiden Elektrodenleitungen (12) und (14) erlauben, während die Längsführungen (18, 18', 18", 18"') in einem zweiten Zustand ein Längsverschieben zwischen den beiden Elektrodenleitungen (12, 14) verhindern und aus diese Weise die beiden Elektrodenleitungen (12, 14) relativ zueinander fixieren.

6. Elektrodenleitung nach Anspruch 5, **dadurch gekennzeichnet, dass** die Längsführungen (18, 18', 18", 18''') Ösen umfassen, die zur längsverschieblichen Aufnahme einer zweiten Elektrodenleitung (14) ausgebildet sind.

7. Elektrodenleitung nach Anspruch 5 oder 6, **dadurch gekennzeichnet, dass** die Längsführungen (18, 18', 18", 18''') Nitinol enthalten.

8. Elektrodenleitung nach einem der Ansprüche 5 bis 7, **dadurch gekennzeichnet, dass** die Längsführungen (18, 18', 18", 18"') in Längsrichtung zwei Enden aufweisen, von denen zumindest eines der Enden eine Schräge (44) aufweist, die ausgehend von der Elektrodenleitung (12, 14) zu dem jeweils anderen Ende hin geneigt ist.

9. Elektrodenleitung nach Anspruch des 8, **dadurch gekennzeichnet, dass** die Schräge (44) von einer offenen Gitterstruktur oder einem Drahtgeflecht gebildet ist.

10. Elektrodenleitung nach einem der Ansprüche 5 bis 9, **dadurch gekennzeichnet, dass** die Längsführungen (18, 18', 18", 18''') auf eine Hülle (16) der Elektrodenleitung (12, 12', 12", 12"') nachträglich aufgebracht sind.

11. Elektrodenleitung nach Anspruch 10, **dadurch gekennzeichnet, dass** die Längsführungen (18, 18', 18", 18"') reibungsvermindernde Zungen (52) aufweisen, die zwischen einem zur Aufnahme einer zweiten Elektrodenleitung (14) vorgesehenen Hohlraum der Längsführungen (18, 18', 18", 18''') und einer Hülle (16) der ersten Elektrodenleitung (12, 12', 12", 12''') angeordnet und derart ausgebildet sind, dass eine Hülle (32) der zweiten Elektrodenleitung (14) auf Grund der Zungen (52) einen Abstand von der Hülle (16) der ersten Elektrodenleitung (12, 12', 12", 12''') einnimmt.

12. Elektrodenleitung nach einem der Ansprüche 5 bis 9, **dadurch gekennzeichnet, dass** die Längsführungen (18, 18', 18", 18"') integraler Bestandteil einer Hülle (16) der Elektrodenleitung (12, 12', 12", 12''') sind.

13. Elektrodenleitung nach einem der Ansprüche 5 bis 12 mit einer Spitzenelektrode (24), **dadurch gekennzeichnet, dass** die Spitzenelektrode (24) in derjenigen Umfangsrichtung der Elektrodenleitung (12, 14) isoliert ist, in die auch die Längsführungen (18, 18', 18", 18"') weisen.

14. Elektrodenleitung nach einem der Ansprüche 5 bis 13, **dadurch gekennzeichnet, dass** wenigstens eine der Längsführungen (18, 18', 18", 18"') bezüglich der Elektrodenleitung (12) derart schwenk- oder kippbar ausgeführt ist, dass es im gekippten bzw. geschwenkten Zustand der Längsführung (18, 18', 18", 18"') zu einem Verklemmen mit einer in der Längsführung befindlichen zweiten Elektrodenleitung (14) kommt.

15. Elektrodenleitung nach einem der Ansprüche 5 bis 13, **dadurch gekennzeichnet, dass** die Elektrodenleitung im Bereich wenigstens einer ihrer Längsführungen in Längsachsenrichtung vorzugsweise S-förmig vorgebogen ist

## Claims

1. Intravascular electrode arrangement (10) comprising a first and a second electrode line (12, 14) adapted for placement in blood vessels such as veins or arteries, each having a stimulation and/or sensing electrode in the region of the distal end of the respective electrode line, and having longitudinal guides (18, 18', 18", 18"') disposed externally on the first electrode line (12, 12', 12", 12''') for the second electrode line (14), **characterised in that** the longitudinal guides (18, 18', 18", 18"') may be manipulated such that, in a first state, they allow longitudinal displacement between the electrode line (12) and a second electrode line (14), whereas, in a second state, the longitudinal guides (18, 18', 18", 18"') prevent longitudinal displacement between the two electrode lines (12, 14) and thus fix the two electrode lines (12, 14) relative to each other.

2. Electrode arrangement according to claim 1, **characterised in that** the longitudinal guides (18, 18', 18", 18''') comprise eyes having an inside diameter that is adapted to the outside diameter of the second electrode line (14) and substantially corresponds thereto.

3. Electrode arrangement according to claim 1, **characterised in that** the outside diameter of the first and second electrode lines (12,14) is less than 2 mm.

4. Electrode arrangement according to claim 1, **characterised in that** the first electrode line (12, 12', 12", 12''') is configured as an atrial electrode catheter and **in that** the second electrode line (14) is configured as a ventricular electrode catheter.

5. Electrode line for an electrode arrangement according to any one of claims 1 to 5, **characterised by** longitudinal guides (18, 18', 18", 18"') disposed externally on the electrode line (12, 14), which longitudinal guides may be manipulated such that, in a first state, they allow longitudinal displacement between the electrode line (12) and a second electrode line (14), whereas, in a second state, the longitudinal guides (18, 18', 18", 18''') prevent longitudinal displacement between the two electrode lines (12, 14) and thus fix the two electrode lines (12, 14) relative to each other.

6. Electrode line according to claim 5, **characterised in that** the longitudinal guides (18, 18', 18", 18"') comprise eyes, which are configured for receiving a second electrode line (14) in a longitudinally displaceable manner.

7. Electrode line according to either claim 5 or claim 6, **characterised in that** the longitudinal guides (18, 18', 18", 18"') contain nitinol.

8. Electrode line according to any one of claims 5 to 7, **characterised in that**, in the longitudinal direction, the longitudinal guides (18, 18', 18", 18''') comprise two ends, at least one of which has a slope (44), which is inclined, starting from the electrode line (12, 14), toward the respective other end.

9. Electrode line according to claim 8, **characterised in that** the sloped end (44) is formed by an open lattice structure or a wire mesh.

10. Electrode line according to any one of claims 5 to 9, **characterised in that** the longitudinal guides (18, 18', 18", 18"') are subsequently attached to a casing (16) of the electrode line (12, 12', 12", 12''').

11. Electrode line according to claim 10, **characterised in that** the longitudinal guides (18, 18', 18", 18"') have friction-reducing tongues (52), which are arranged between a hollow space in the longitudinal guides (18, 18', 18", 18'''), which is provided to receive a second electrode line (14), and a casing (16) of the first electrode line (12, 12', 12", 12"'), and are configured such that a casing (32) of the second electrode line (14) is set apart from the casing (16) of the first electrode line (12,12', 12", 12"') as a result of the tongues (52).

12. Electrode line according to any one of claims 5 to 9, **characterised in that** the longitudinal guides (18, 18', 18", 18"') are an integral part of a casing (16) of the electrode line (12, 12', 12", 12''').

13. Electrode line according to any one of claims 5 to 12 comprising a tip electrode (24), **characterised in that** the tip electrode (24) is insulated in the peripheral direction of the electrode line (12, 14) in which the longitudinal guides (18,18', 18", 18"') also face.

14. Electrode line according to any one of claims 5 to 13, **characterised in that** at least one of the longitudinal guides (18, 18', 18", 18"') is pivotable or tiltable with respect to the electrode line (12) such that, in the tilted or pivoted state of the longitudinal guide (18, 18', 18", 18"'), a second electrode line (14) located therein is clamped.

15. Electrode line according to any one of claims 5 to 13, **characterised in that** the electrode line, in the region of at least one of its longitudinal guides, is pre-bent in the direction of the longitudinal axis, preferably in the shape of an 'S'.

## Revendications

1. Ensemble d'électrodes intravasculaire (10) comprenant une première et une deuxième conduites d'électrodes (12, 14) adaptées pour le placement dans des vaisseaux sanguins comme des veines ou des artères ayant chacune au moins une électrode de stimulation et/ou de détection dans la zone de l'extrémité distale de la conduite d'électrode respective, ainsi que des guides longitudinaux (18, 18', 18", 18"') installés à l'extérieur sur la première conduite d'électrode (12, 12', 12", 12"') pour la deuxième conduite d'électrode (14), **caractérisé en ce que** les guides longitudinaux (18, 18', 18", 18"') sont conçus pour être manipulables de telle sorte qu'ils permettent dans une première position un coulissement longitudinal entre la conduite d'électrode (12) et une deuxième conduite d'électrode (14), tandis que les guides longitudinaux (18, 18', 18", 18''') empêchent dans une deuxième position un coulissement longitudinal entre les deux conduites d'électrodes (12, 14) et fixent de cette manière les deux conduites d'électrodes (12, 14) l'une par rapport à l'autre.

2. Ensemble d'électrodes selon la revendication 1, **caractérisé en ce que** les guides longitudinaux (18, 18', 18", 18"') comprennent des boucles ayant un diamètre intérieur qui est adapté au diamètre extérieur de la deuxième conduite d'électrode (14) et correspond pour l'essentiel à celui-ci.

3. Ensemble d'électrodes selon la revendication 1, **caractérisé en ce que** le diamètre extérieur des première et deuxième conduites d'électrodes (12, 14) est inférieur à 2 mm.

4. Ensemble d'électrodes selon la revendication 1, **caractérisé en ce que** la première conduite d'électrode (12, 12', 12", 12''') est réalisée en tant que cathéter d'électrode pour oreillette et **en ce que** la deuxième conduite d'électrode (14) est réalisée en tant que cathéter d'électrode pour ventricule.

5. Conduite d'électrode pour un ensemble d'électrode selon l'une quelconque des revendications 1 à 4, **caractérisée par** des guides longitudinaux (18, 18', 18", 18''') installés à l'extérieur sur la conduite d'électrode (12, 14) qui sont conçus pour être manipulables de telle sorte qu'ils permettent dans une première position un coulissement longitudinal entre la conduite d'électrode (12) et une deuxième conduite d'électrode (14), tandis que les guides longitudinaux (18, 18', 18", 18''') empêchent dans une deuxième position un coulissement longitudinal entre les deux conduites d'électrodes (12, 14) et fixent de cette manière les deux conduites d'électrodes (12, 14) l'une par rapport à l'autre.

6. Conduite d'électrode selon la revendication 5, **caractérisée en ce que** les guides longitudinaux (18, 18', 18", 18"') comprennent des boucles qui sont conçues pour recevoir dans un coulissement longitudinal une deuxième conduite d'électrode (14).

7. Conduite d'électrode selon l'une quelconque des revendications 5 ou 6, **caractérisée en ce que** les guides longitudinaux (18, 18', 18", 18"') contiennent du nitinol.

8. Conduite d'électrode selon l'une quelconque des revendications 5 à 7, **caractérisée en ce que** les guides longitudinaux (18, 18', 18", 18''') présentent dans la direction longitudinale deux extrémités, parmi lesquelles au moins l'une des extrémités présente un biseau (44) qui est incliné à partir de la conduite d'électrode (12, 14) en direction de l'autre extrémité respective.

9. Conduite d'électrode selon la revendication 8, **caractérisée en ce que** le biseau (44) est formé par une structure réticulaire ouverte ou un treillis en fil métallique.

10. Conduite d'électrode selon l'une quelconque des revendications 5 à 9, **caractérisée en ce que** les guides longitudinaux (18, 18', 18", 18"') sont installés durablement sur une enveloppe (16) de la conduite d'électrode (12, 12', 12", 12''').

11. Conduite d'électrode selon la revendications 10, **caractérisée en ce que** les guides longitudinaux (18, 18', 18", 18"') présentent des languettes atténuant le frottement (52), qui sont agencées entre un espace creux des guides longitudinaux (18, 18', 18", 18"') prévu pour recevoir une deuxième conduite d'électrode (14) et une enveloppe (16) de la première conduite d'électrode (12, 12', 12", 12"') et sont conçues de telle sorte qu'une enveloppe (32) de la deuxième conduite d'électrode (14) maintient une distance par rapport à l'enveloppe (16) de la première conduite d'électrode (12, 12', 12'', 12''').

12. Conduite d'électrode selon l'une quelconque des revendications 5 à 9, **caractérisée en ce que** les guides longitudinaux (18, 18', 18", 18''') font partie intégrante d'une enveloppe (16) de la conduite d'électrode (12, 12', 12", 12''').

13. Conduite d'électrode selon l'une quelconque des revendications 5 à 12 ayant une électrode à pointe (24), **caractérisée en ce que** l'électrode à pointe (24) est isolée dans la direction de la périphérie de la conduite d'électrode (12, 14) dans laquelle sont également orientés les guides longitudinaux (18, 18', 18", 18''').

14. Conduite d'électrode selon l'une quelconque des revendications 5 à 13, **caractérisée en ce qu'**au moins l'un des guides longitudinaux (18, 18', 18", 18''') est réalisé de façon à pouvoir pivoter ou basculer par rapport à la conduite d'électrode (12) de telle sorte qu'il se produit dans la position basculée ou pivotée du guide longitudinal (18, 18', 18", 18"') un serrage avec une deuxième conduite d'électrode (14) se trouvant dans le guide longitudinal.

15. Conduite d'électrode selon l'une quelconque des revendications 5 à 13, **caractérisée en ce que** la conduite d'électrode est précourbée dans la direction de son axe longitudinal de préférence en forme de S dans la zone d'au moins l'un de ses guides longitudinaux.
